# EUROPEAN PATENT APPLICATION

(11) **EP 1 759 630 A2**
(43) Date of publication of application: **07.03.2007**
(21) Application number: 06018102.1
(22) Date of filing: 30.08.2006
(51) Int. Cl.: A61B 3/06

(54) **Ophthalmic examination program, ophthalmic examination apparatus, and ophthalmic examination system**

(30) Priority: 02.09.2005 JP 2005255185
(71) Applicant: Kabushiki Kaisha TOPCON, Tokyo 174-8580 (JP)
(72) Inventor: Noda, Toru, Tokyo 152-8902 (JP); Ooyagi, Wataru, Itabashi-ku, Tokyo 174-8580 (JP); Shibutani, Masahiro, Itabashi-ku, Tokyo 174-8580 (JP)
(74) Representative: Vossius & Partner

(57) **Abstract**

A simulation image of an index image formed on a fundus of an eye to be examined when each of a plurality of indices is presented to the eye to be examined is generated based on optical characteristic data of the eye to be examined and image data of the indices associated with respective spatial frequencies. Of the generated simulation images, simulation images whose contrasts can be recognized by the eye to be examined are selected at each of the spatial frequencies. Of the selected simulation images, a simulation image in which a contrast of an index being a basis for the simulation image is minimum is specified. Mark information for marking index information of indices which are the bases for simulation images specified at each of the spatial frequencies are added to image data of an examination sheet and displayed thereon (Fig. 1).

## Description

The present invention relates to an ophthalmic examination program, an ophthalmic examination apparatus, and an ophthalmic examination system which are applied to measure contrast sensitivity of an eye to be examined in an ophthalmic field.

A contrast sensitivity examination is an ophthalmic examination widely performed, for example, before or after a cataract operation, a glaucoma operation, or a laser in situ keratomileusis (LASIK) operation.

When the contrast sensitivity examination is to be performed, a specific index is presented to an eye to be examined such that a reply based on the visibility thereof is made by a person to be examined (that is, this is a subjective ophthalmic examination). Hereinafter, an outline of the contrast sensitivity examination will be described with reference to "Retina Topography and Wavefront Sensor - Point of Decoding" edited by Naoyuki Maeda, Tetsuro Oshika, and Takashi Fujikado, Medical View Co., Ltd, October 10, 2002, pp. 214-222.

The contrast sensitivity examination is performed by presenting a specific index such as a stripe index or an ETDRS chart (including alphabets and Landolt rings) to the eye to be examined. Hereinafter, the contrast sensitivity examination using the stripe index will be described.

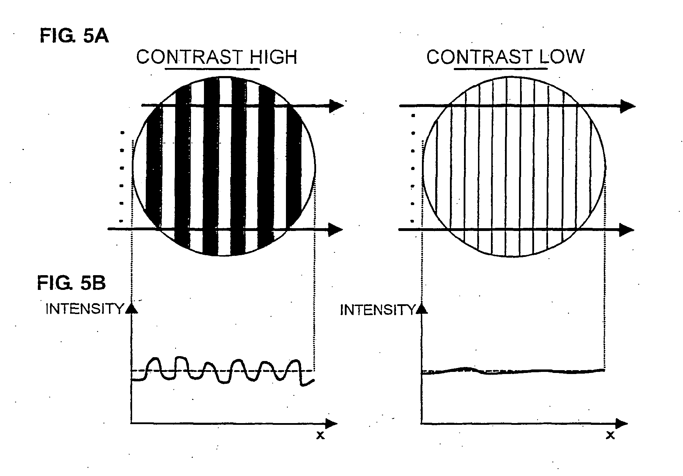

The stripe index is an index having stripes in which a contrast sinusoidally changes. A plurality of stripe indices whose stripe intervals (spatial frequencies) are different from one another and whose contrasts are different from one another are prepared for the examination. The stripe indices are presented to the eye to be examined in order from a stripe index whose contrast is highest such that a reply based on a visibility thereof is made by a person to be examined. Therefore, an index whose contrast is minimum, of indices in which the person to be examined can recognize the presence of the stripe at each of the spatial frequencies is specified as a result obtained by examination. The result is entered on a sheet as shown in FIG 5 of "Retina Topography and Wavefront Sensor - Point of Decoding" edited by Naoyuki Maeda, Tetsuro Oshika, and Takashi Fujikado, Medical View Co., Ltd, October 10, 2002. In this sheet, an abscissa takes a spatial frequency and an ordinate takes contrast sensitivity (a reciprocal of the contrast). The sheet shows a normal range of the contrast sensitivity, thereby facilitating judgement on whether the result is normal or abnormal.

An ophthalmic apparatus described in JP-A-6-165754 and a visual function examination apparatus described in JP-A-2002-191558 are conventional inventions related to the contrast sensitivity examination.

The ophthalmic apparatus described in JP-A-6-165754 includes a stripe pattern generating and displaying means, a reply inputting means, a result displaying means, and a signal processing and control means. The stripe pattern generating and displaying means successively presents stripe patterns (stripe indices) to the eye to be examined in a preprogrammed sequence. The signal processing and control means determines contrast sensitivity based on input information from the reply inputting means. The result displaying means displays contrast sensitivity. Therefore, even when there is no examiners, the contrast sensitivity examination can be performed. The stripe pattern generating and displaying means can present the stripe patterns to the eye to be examined while a pitch of the stripes or a direction thereof are changed in random, thereby improving objectivity of measurement data.

The visual function examination apparatus described in JP-A-2002-191558 is a space-saving visual function examination apparatus. In this apparatus, an index light flux from an index presenting means is reflected on a mirror surface and then reflected by a beam splitter whose index light side is obliquely provided in the rear of an opening portion of a case. After that, the light flux travels to the eye to be examined which is located at a distance from the case. The visual function examination apparatus includes an illumination means for a contrast test, a combining mirror, and a light amount ratio changing means. The illumination means includes an illumination light source for changing contrast and a diffusing plate for diffusing illumination light from the illumination light source. The combining mirror has a characteristic of reflecting a part of a light flux in the visible region and transmitting a part thereof and is used to combine the index light flux with the illumination light flux. The light amount ratio changing means changes a light amount ratio between the amount of illumination light from the illumination means for a contrast test and an amount of index light flux based on a predetermined contrast ratio. Therefore, various examinations including the contrast sensitivity examination can be performed by a single apparatus, thereby achieving space saving.

In recent years, an advance of a technique for measuring optical characteristic data of the eye to be examined and simulating sizes of projection images of indices or the like which are projected to a fundus (retina) of the eye to be examined is being made (see, for example, JP-A-2002-209852 and JP-A-2004-337236 related to the applicant of the present invention).

In an apparatus described in JP-A-2002-209852, a plurality of images are obtained while a focusing state of a light flux for illuminating the eye to be examined is changed. A point spread function (PSF) of the eye to be examined is calculated based on the obtained images. The PSF and index image data (intensity distribution) are convolved with each other (convolution is performed) to obtain a simulation image in the case where the index is projected to the fundus of the eye to be examined.

In order to shorten a calculation time for obtaining the simulation image, a following calculation method can be used. First, the PSF is subjected to Fourier transform to obtain an optical transfer function (OTF). An absolute value component of an amplitude of the OTF is called a modulation transfer function (MTF) and a phase component thereof is called a phase transfer function (PTF). The MTF is convolved with the index image data and the PTF is convolved with the index image data. Then, a result obtained by those convolutions is subjected to inverse Fourier transform to obtain the simulation image corresponding to the convolution of the PSF and the index image data.

In an apparatus described in JP-A-2004-337236, a light flux with which the eye to be examined is illuminated is received through a Hartmann plate or the like. A pupil diameter of the eye to be examined, total wavefront aberration, Zernike coefficients, and the like are calculated based on a received signal. A pupil function is obtained based on a result obtained by calculation. An OTF of an eyeball of the eye to be examined is obtained based on the obtained pupil function. Image data of an index is subjected to Fourier transform to obtain a spatial frequency distribution thereof. The spatial frequency distribution of the index image data is convolved with the OTF of the eyeball of the eye to be examined to obtain a frequency distribution of light passing through an eyeball optical system. The obtained frequency distribution is subjected to inverse Fourier transform to obtain a simulation image corresponding to an eye fundus projection image of the index.

As described above, the contrast sensitivity examination is the subjective examination performed by successively presenting a large number of stripe indices whose contrasts are different from one another and spatial frequencies are different from one another to the eye to be examined. In this examination, for example, eight stripe indices whose contrasts are different from one another are prepared for each of four kinds of spatial frequency values. Therefore, it is necessary that an index presenting operation and a reply operation performed by the person to be examined be repeated 32 times in maximum. Thus, the contrast sensitivity examination is an examination which is essential, for example, before or after an operation but requires much time, so that a burden on both the examiner and the person to be examined is large.

The contrast sensitivity examination is the subjective examination for obtaining a subjective result based on a reply from the person to be examined, so it is hard to say that the objectivity of the result obtained by the examination is sufficiently ensured.

Under such a situation, according to the invention described in JP-A-6-165754, the contrast sensitivity examination can be performed by the person to be examined alone. However, a burden on the person to be examined does not reduce. Conversely, the examiner cannot provide support, so the burden on the person to be examined may be increased by just that much.

In addition, according to the invention described in JP-A-6-165754, the indices are presented while the pitch of the stripes or the direction thereof are changed in random, thereby improving the objectivity of the result obtained by examination. In any case, the subjective examination method of obtaining a result based on reply information from the person to be examined is employed, so the above-mentioned presentation does not provide support for essentially improving the objectivity of the result obtained by examination.

Similarly, even in the case of the invention described in JP-A-2002-191558, the subjective examination method is employed, so it is difficult to solve the problems with respect to the objectivity of the result obtained by the examination.

The present invention has been made to solve the above-mentioned problems. An object of the present invention is to provide an ophthalmic examination program, an ophthalmic examination apparatus, and an ophthalmic examination system which can improve objectivity of a result obtained by a contrast sensitivity examination.

Another object of the present invention is to provide an ophthalmic examination program, an ophthalmic examination apparatus, and an ophthalmic examination system which can shorten an examination time of a contrast sensitivity examination.

According to a first aspect of the present invention, there is provided an ophthalmic examination program for operating a computer including storing means for prestoring image data of a predetermined examination sheet in which index information indicating a plurality of indices whose contrasts are different from one another at each of at least two different spatial frequencies and image data of the plurality of indices associated with the at least two spatial frequencies are arranged and display means,
in which the ophthalmic examination program is characterized in causing the computer to function as:
image generating means for generating a simulation image of an index image formed on a fundus of an eye to be examined when each of the plurality of indices is presented to the eye to be examined is generated based on optical characteristic data of the eye to be examined and image data of the plurality of indices associated with the at least two spatial frequencies,
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images,
examination result creating means for adding mark information for making index information of the index which is the basis for the specified simulation image onto the image data of the predetermined examination sheet; and
examination result display control means for causing the display means to display the image data of the examination sheet to which the mark information is added.

According to a second aspect of the present invention, there is provided an ophthalmic examination apparatus connected to an eye's optical characteristic measuring apparatus for obtaining optical characteristic data of an eye to be examined, in which the ophthalmic examination apparatus is characterized in including:
storing means for prestoring image data of a predetermined examination sheet in which index information indicating a plurality of indices whose contrasts are different from one another at each of at least two different spatial frequencies and image data of the plurality of indices associated with the at least two spatial frequencies are arranged;
display means;
image generating means for generating a simulation image of an index image formed on a fundus of an eye to be examined when each of the plurality of indices is presented to the eye to be examined is generated based on optical characteristic data of the eye to be examined and image data of the plurality of indices associated with the at least two spatial frequencies;
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images;
examination result creating means for adding mark information for making index information of the index which is the basis for the specified simulation image onto the image data of the predetermined examination sheet; and
examination result display control means for causing the display means to display the image data of the predetermined examination sheet to which the mark information is added.

According to a third aspect of the present invention, there is provided an ophthalmic examination system, including:
an eye's ophthalmic characteristic measuring apparatus for obtaining optical characteristic data; and
an ophthalmic examination apparatus,
in which the ophthalmic examination apparatus is characterized in including:
storing means for prestoring image data of a predetermined examination sheet in which index information indicating a plurality of indices whose contrasts are different from one another at each of at least two different spatial frequencies and image data of the plurality of indices associated with the at least two spatial frequencies are arranged;
display means;
image generating means for generating a simulation image of an index image formed on a fundus of an eye to be examined when each of the plurality of indices is presented to the eye to be examined is generated based on optical characteristic data, obtained by the eye's optical characteristic measuring apparatus, of the eye to be examined and image data of the plurality of indices associated with the at least two spatial frequencies;
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images;
examination result creating means for adding mark information for making index information of the index which is the basis for the specified simulation image onto the image data of the predetermined examination sheet; and
examination result display control means for causing the display means to display the image data of the predetermined examination sheet to which the mark information is added.

According to the present invention, the simulation image of the index image formed on the fundus of the eye to be examined when each of the plurality of indices is presented to the eye to be examined can be generated based on the optical characteristic data of the eye to be examined and the image data of the indices associated with respective spatial frequencies. Of the generated simulation images, the simulation images whose contrasts can be recognized by the eye to be examined are selected at each of the spatial frequencies. Of the selected simulation images, a simulation image in which the contrast of the index which is the basis for the simulation image is minimum can be specified. The mark information for marking the index information of the indices which are the bases for the simulation images specified at each of the spatial frequencies can be added to the image data of the examination sheet and displayed thereon.

Therefore, when the simulation images with respect to the visibility of the indices which are based on the optical characteristic data of the eye to be examined are used, the contrast sensitivity of the eye to be examined can be objectively examined. Thus, an objectivity of a result obtained by a contrast sensitivity examination can be improved as compared with a conventional subjective examination.

Further, according to the present invention, when the contrast sensitivity examination is performed, it is unnecessary that each of a large number of indices be presented to the eye to be examined one by one to receive a reply from a person to be examined, unlike a conventional case. Thus, an examination time can be shortened.

In the accompanying drawings:
FIG. 1 is a schematic block diagram showing an example of an entire structure of an ophthalmic examination system according to a first embodiment of the present invention;
FIG. 2 is a schematic block diagram showing an example of a hardware structure of an ophthalmic examination apparatus included in the ophthalmic examination system according to the first embodiment of the present invention;
FIG. 3 is a schematic explanatory diagram for explaining an outline of an example of examination sheet data stored in the ophthalmic examination apparatus included in the ophthalmic examination system according to the first embodiment of the present invention;
FIG. 4 is a schematic explanatory diagram for explaining an outline of an example of index data stored in the ophthalmic examination apparatus included in the ophthalmic examination system according to the first embodiment of the present invention;
FIGS. 5A and 5B are schematic explanatory diagrams for explaining an example of image specifying processing performed by the ophthalmic examination apparatus included in the ophthalmic examination system according to the first embodiment of the present invention;
FIG. 6 is a schematic explanatory diagram for explaining an example of mark information added to the examination sheet data by the ophthalmic examination apparatus included in the ophthalmic examination system according to the first embodiment of the present invention;
FIG. 7 is a schematic explanatory diagram for explaining an example of a graph added to the examination sheet data by the ophthalmic examination apparatus included in the ophthalmic examination system according to the first embodiment of the present invention;
FIG. 8 is a schematic explanatory diagram for explaining an example of a graph added to the examination sheet data by the ophthalmic examination apparatus included in the ophthalmic examination system according to the first embodiment of the present invention;
FIG. 9 is a schematic explanatory diagram for explaining an example of a region on the examination sheet data which becomes a calculation target of an area (AULCSF) calculated by the ophthalmic examination apparatus included in the ophthalmic examination system according to the first embodiment of the present invention;
FIG. 10 is a schematic explanatory diagram for explaining an example of a display pattern of an examination result obtained by the ophthalmic examination apparatus included in the ophthalmic examination system according to the first embodiment of the present invention;
FIG. 11 is a flow chart for explaining an example of an operation of the ophthalmic examination system according to the first embodiment of the present invention;
FIG. 12 is a schematic block diagram showing an example of the entire structure of an ophthalmic examination system according to a second embodiment of the present invention; and
FIG. 13 is a flow chart for explaining an example of an operation of the ophthalmic examination system according to the second embodiment of the present invention.

Examples of an ophthalmic examination program, an ophthalmic examination apparatus, and an ophthalmic examination system according to preferred embodiments of the present invention will be described in detail with reference to the accompany drawings.

### <First Embodiment>

### [System Structure]

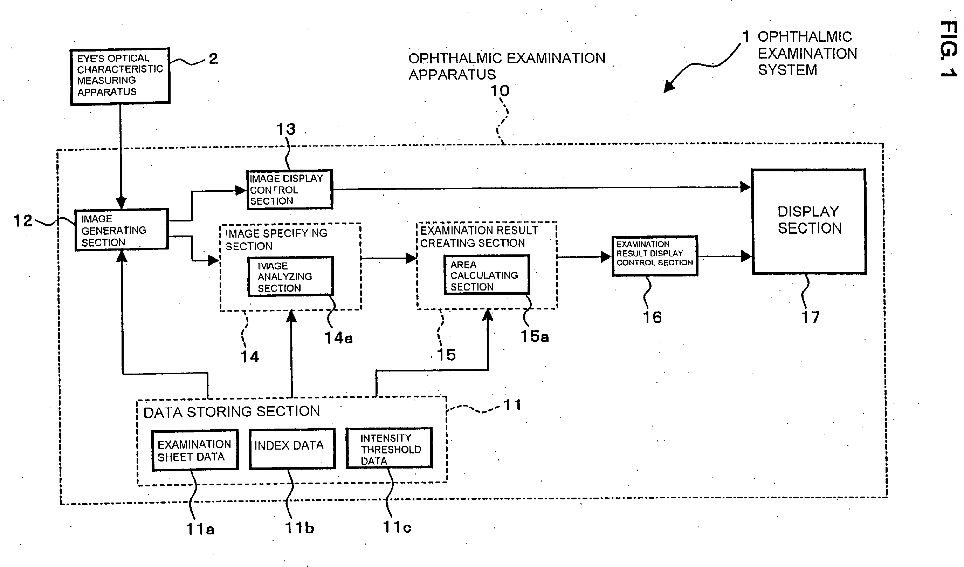

FIG. 1 shows an example of an entire structure of an ophthalmic examination system according to this embodiment. An ophthalmic examination system 1 shown in FIG 1 includes an eye's optical characteristic measuring apparatus 2 and an ophthalmic examination apparatus 10.

### [Eye's Optical Characteristic Measuring Apparatus]

The eye's optical characteristic measuring apparatus 2 includes, for example, an optical device for measuring eye's optical characteristics of an eye to be examined as described in JP-A-2002-209852 or JP-A-2004-337236. Assume that the "eye's optical characteristics" means data indicating optical characteristics of the eye to be examined, such as a modulation transfer function (MTF), a phase transfer function (PTF), and a point spread function (PSF).

### [Ophthalmic Examination Apparatus]

The ophthalmic examination apparatus 10 corresponds to an example of the "ophthalmic examination apparatus" according to the present invention and includes a computer used to perform a contrast sensitivity examination of the eye to be examined. As shown in FIG 1, the ophthalmic examination apparatus 10 includes a data storing section 11, an image generating section 12, an image display control section 13, an image specifying section 14, an examination result creating section 15, an examination result display control section 16, and a display section 17.

### [Hardware Structure]

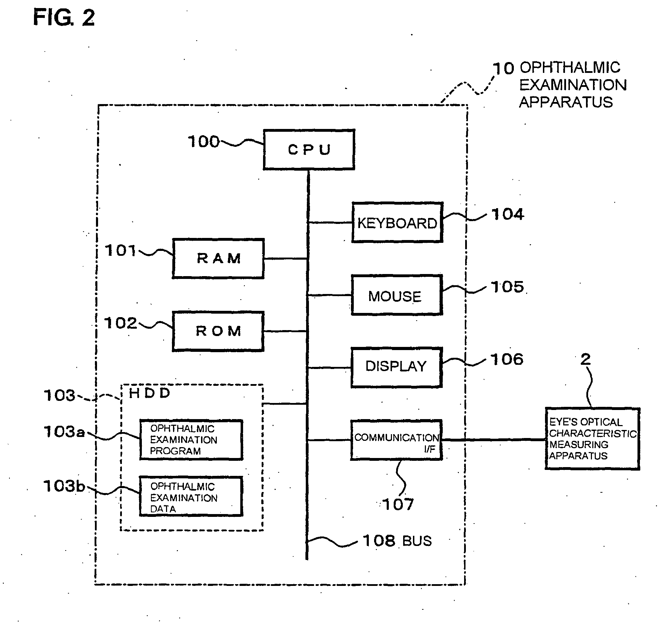

FIG. 2 shows an example of a hardware structure of the ophthalmic examination apparatus 10 which is necessary to realize functions of the above-mentioned respective sections which will be described later. Before functional structures of the respective sections are described, the hardware structure of the ophthalmic examination apparatus 10 will be described.

The ophthalmic examination apparatus 10 has the same hardware structure as that of a conventional computer. To be specific, the ophthalmic examination apparatus 10 includes (a microprocessor such as) a CPU 100, a RAM 101, a ROM 102, a hard disk drive (HDD) 103, a keyboard 104, a mouse 105, a display 106, and a communication interface (I/F) 107. The respective sections are connected with one another through a bus 108.

The CPU 100 loads, into the RAM 101, an ophthalmic examination program 103a and an ophthalmic examination data 103b which are stored in the hard disk drive 103 and then executes a typical operation of the present invention as described later. The CPU 100 also executes control of the respective sections of the apparatus, various calculation processings, and the like. For example, the CPU 100 controls the respective sections of the apparatus according to operating signals from the keyboard 104 or the mouse 105, controls display processing for the display 106, and controls data communication processing using the communication interface 107.

The ophthalmic examination program 103a and the ophthalmic examination data 103b are stored in the hard disk drive 103 in advance. The ophthalmic examination program 103a is a program for causing the ophthalmic examination apparatus 10 having the hardware structure shown in FIG. 2 to execute an operation (described later) of the respective sections shown in FIG 1. The ophthalmic examination data 103b includes various data used for processing based on the ophthalmic examination program 103a. Various data such as measurement data of the eye's optical characteristics which are transmitted from the eye's optical characteristic measuring apparatus 2 are stored in the hard disk drive 103 as appropriate.

The keyboard 104 is used as an input device for inputting characters, numerals, and the like by typing. The mouse 105 is used as a manipulating device for performing various input manipulations on a display screen of the display 106 and also used as an input device. The display 106 is an arbitrary display device such as a liquid crystal display (LCD) or a cathode ray tube (CRT).

Each of the keyboard 104, the mouse 105 and the display 106 is used as a user interface of the ophthalmic examination apparatus 10. The display 106 corresponds to an example of the display section 17 shown in FIG. 1.

Each of the user interfaces is not limited to the corresponding structure. For example, each of the user interfaces can include an arbitrary user interface means having a function for displaying and outputting various information and a function for inputting various information, such as a trackball, a joystick, a touch panel LCD, or an ophthalmic examination control panel.

The communication interface 107 receives the data of the eye's optical characteristics measured by the eye's optical characteristic measuring apparatus 2. When the ophthalmic examination apparatus 10 is connected with the eye's optical characteristic measuring apparatus 2 through a local area network (LAN), the communication interface 107 includes a network adapter such as a LAN card. When the ophthalmic examination apparatus 10 is connected with the eye's optical characteristic measuring apparatus 2 through a private line, the communication interface 107 includes a connector for the private line.

### [Functional Structure]

The functional structure of the ophthalmic examination apparatus 10 having the above-mentioned hardware structure will be described with reference to FIGS. 1 and 2. Hereinafter, the respective sections of the ophthalmic examination apparatus 10 shown in FIG. 1 will be described.

### (Data Storing Section)

The data storing section 11 includes an arbitrary storage device (nonvolatile storage device is desirable). To be specific, the data storing section 11 includes the hard disk drive 103 shown in FIG. 2. Examination sheet data 11a, index data 11b, and intensity threshold value data 11c are stored in the data storing section 11 in advance.

The examination sheet data 11 a is image data of a predetermined examination sheet on which a result obtained by the contrast sensitivity examination is to be entered. The index data 11b is image data of indices used for the contrast sensitivity examination. The intensity threshold value data 11c is numeral data used as a threshold value for determining whether or not an index shown on a simulation image can be recognized by the eye to be examined during image specifying processing described later.

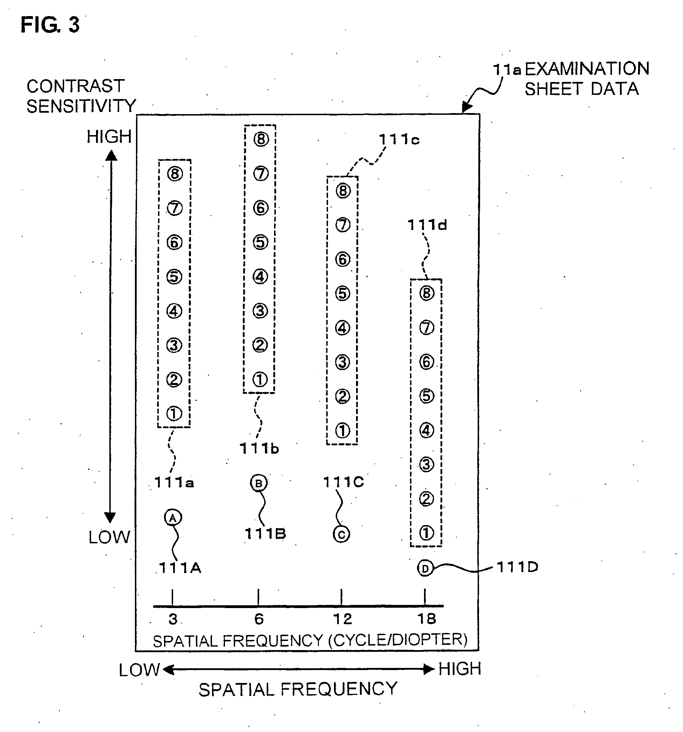

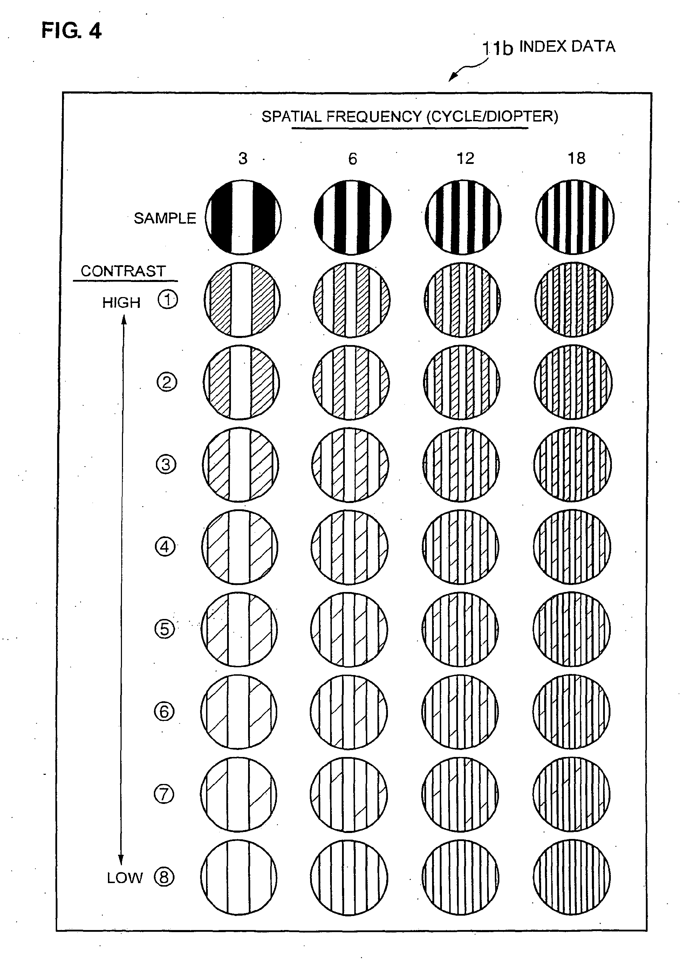

FIG. 3 schematically shows an example of an examination sheet stored in the examination sheet data 11a (see, for example, "Retina Topography and Wavefront Sensor - Point of Decoding" edited by Naoyuki Maeda, Tetsuro Oshika, and Takashi Fujikado, Medical View Co., Ltd, October 10, 2002). FIG 4 schematically shows stripe indices which are examples of contrast sensitivity examination indices which are stored as the index data 11b (see, for example, "Retina Topography and Wavefront Sensor - Point of Decoding" edited by Naoyuki Maeda, Tetsuro Oshika, and Takashi Fujikado, Medical View Co., Ltd, October 10, 2002). A contrast level of each of the stripe indices shown in FIG 4 is expressed according to an oblique line density of regions in which stripes are dense.

The stripe indices as shown in FIG 4 are used for the contrast sensitivity examination performed using the ophthalmic examination system 1. In this embodiment, eight stripe indices whose contrasts are different from one another are used for each of four different spatial frequencies (3, 6, 12, and 18 cycles/diopter). The index data 11b include data indicating intensity distributions of respective images of the 32 stripe indices (intensity distribution data).

In the index data 11b shown in FIG 4, of the stripe indices arranged (in a longitudinal direction of a paper) at each of the spatial frequencies, a stripe index located in the uppermost position is a sample index at each spatial frequency. Eight stripe indices associated with each of the spatial frequencies are located below the corresponding sample index and arranged in order from a stripe index whose contrast is highest.

The eight stripe indices associated with each of the spatial frequencies are provided with index information "circle 1 ", "circle 2", ..., "circle 8" in order from the stripe index whose contrast is highest. The index information is identification information for identifying the respective stripe indices.

Each of the 32 stripe indices has, for example, a contrast (contrast sensitivity) stripe described in "Retina Topography and Wavefront Sensor - Point of Explanation" edited by Naoyuki Maeda, Tetsuro Oshika, and Takashi Fujikado, Medical View Co., Ltd, October 10, 2002. That is, in the case where the spatial frequency is 3 cycles/diopter, a contrast of the sample index is set to 20.00% (contrast sensitivity is 5). A contrast of the stripe index provided with the index information "circle 1" is set to 10.00% (contrast sensitivity is 10). A contrast of the stripe index provided with the index information "circle 2" is set to 6.67% (contrast sensitivity is 15). A contrast of the stripe index provided with the index information "circle 3" is set to 4.55% (contrast sensitivity is 22). A contrast of the stripe index provided with the index information "circle 4" is set to 3.23% (contrast sensitivity is 31). A contrast of the stripe index provided with the index information "circle 5" is set to 2.33% (contrast sensitivity is 43). A contrast of the stripe index provided with the index information "circle 6" is set to 1.64% (contrast sensitivity is 61). A contrast of the stripe index provided with the index information "circle 7" is set to 1.18% (contrast sensitivity is 85). A contrast of the stripe index provided with the index information "circle 8" is set to 0.83% (contrast sensitivity is 120).

In a case where the spatial frequency is 6 cycles/diopter, a contrast of the sample index is set to 12.50% (contrast sensitivity is 8). A contrast of the stripe index provided with the index information "circle 1" is set to 6.25% (contrast sensitivity is 16). A contrast of the stripe index provided with the index information "circle 2" is set to 4.17% (contrast sensitivity is 24). A contrast of the stripe index provided with the index information "circle 3" is set to 2.78% (contrast sensitivity is 36). A contrast of the stripe index provided with the index information "circle 4" is set to 2.00% (contrast sensitivity is 50). A contrast of the stripe index provided with the index information "circle 5" is set to 1.43% (contrast sensitivity is 70). A contrast of the stripe index provided with the index information "circle 6" is set to 1.01% (contrast sensitivity is 99). A contrast of the stripe index provided with the index information "circle 7" is set to 0.72% (contrast sensitivity is 138). A contrast of the stripe index provided with the index information "circle 8" is set to 0.52% (contrast sensitivity is 193).

In a case where the spatial frequency is 12 cycles/diopter, a contrast of the sample index is set to 25.00% (contrast sensitivity is 4). A contrast of the stripe index provided with the index information "circle 1" is set to 12.50% (contrast sensitivity is 8). A contrast of the stripe index provided with the index information "circle 2" is set to 8.33% (contrast sensitivity is 12). A contrast of the stripe index provided with the index information "circle 3" is set to 5.56% (contrast sensitivity is 18). A contrast of the stripe index provided with the index information "circle 4" is set to 4.00% (contrast sensitivity is 25). A contrast of the stripe index provided with the index information "circle 5" is set to 2.86% (contrast sensitivity is 35). A contrast of the stripe index provided with the index information "circle 6" is set to 2.00% (contrast sensitivity is 50). A contrast of the stripe index provided with the index information "circle 7" is set to 1.43% (contrast sensitivity is 70). A contrast of the stripe index provided with the index information "circle 8" is set to 1.01% (contrast sensitivity is 99).

In a case where the spatial frequency is 18 cycles/diopter, a contrast of the sample index is set to 66.66% (contrast sensitivity is 1.5). A contrast of the stripe index provided with the index information "circle 1" is set to 33.33% (contrast sensitivity is 3). A contrast of the stripe index provided with the index information "circle 2" is set to 22.22% (contrast sensitivity is 4.5). A contrast of the stripe index provided with the index information "circle 3" is set to 14.29% (contrast sensitivity is 7). A contrast of the stripe index provided with the index information "circle 4" is set to 10.53% (contrast sensitivity is 9.5). A contrast of the stripe index provided with the index information "circle 5" is set to 7.69% (contrast sensitivity is 13). A contrast of the stripe index provided with the index information "circle 6" is set to 5.56% (contrast sensitivity is 18). A contrast of the stripe index provided with the index information "circle 7" is set to 4.00% (contrast sensitivity is 25). A contrast of the stripe index provided with the index information "circle 8" is set to 2.78% (contrast sensitivity is 36).

The examination sheet data 11 a shown in FIG. 3 is image data of an examination sheet on which a result obtained by examination using the 32 stripe indices shown in FIG. 4 is to be entered. Therefore, the examination sheet data 11a includes information to be entered, indicating whether or not eight stripe indices "circle 1" to "circle 8" associated with each of the spatial frequencies (3, 6, 12, and 18 cycles/diopter) are recognized by the eye to be examined.

To be specific, in the case of the spatial frequency of 3 cycles/diopter, arranged in the examination sheet data 11a shown in FIG 3 are index information "circle A" of the sample index (reference symbol 111A in FIG. 3) and index information "circle 1" to "circle 8" of the eight stripe indices whose contrasts are different from one another (reference symbol 111a). Of the eight stripe indices associated with the index information 111a, a stripe index whose contrast sensitivity is high is located on an upper side of FIG. 3 and a stripe index whose contrast sensitivity is low is located on a lower side of FIG. 3. In other words, of the eight stripe indices associated with the index information 111a, a stripe index whose contrast is low is located on the upper side of FIG. 3 and a stripe index whose contrast is high is located on the lower side of FIG. 3. An arrangement order of the eight stripe indices shown in FIG. 3 is opposite to an arrangement order of the eight stripe indices shown in FIG. 4. The same is expected even in the cases where the respective spatial frequencies are 6, 12, and 18 cycles/diopter as described later.

Similarly, in the case of the spatial frequency of 6 cycles/diopter, arranged in the examination sheet data 11a are index information "circle B" of the sample index (reference symbol 111B in FIG. 3) and index information "circle 1" to "circle 8" of the eight stripe indices whose contrasts (contrast sensitivities) are different from one another (reference symbol 111b). In the case of the spatial frequency of 12 cycles/diopter, index information "circle C" of the sample index (reference symbol 111C in FIG. 3) and index information "circle 1" to "circle 8" of the eight stripe indices whose contrasts (contrast sensitivities) are different from one another (reference symbol 111c) are arranged. In the case of the spatial frequency of 18 cycles/diopter, index information "circle D" of the sample index (reference symbol 111D in FIG. 3) and index information "circle 1" to "circle 8" of the eight stripe indices whose contrasts (contrast sensitivities) are different from one another (reference symbol 111 d) are arranged.

The above-mentioned data storing section 11 corresponds to an example of "storing means" in the present invention, in which the image data of the examination sheet (examination sheet data 11a) and the image data of the indices (index data 11b) are stored in advance.

### (Image Generating Section)

The image generating section 12 corresponds to an example of "image generating means" in the present invention, for generating a simulation image of an index image formed on the fundus of the eye to be examined when each of the 32 stripe indices indicated by the index data 11b is presented to the eye to be examined, based on the data of the eye's optical characteristics of the eye to be examined and the index data 11b (intensity distribution data of the stripe indices) stored in the data storing section 11. The image generating section 12 is constructed by including the CPU 100 shown in FIG. 2.

An example of processing details of simulation image generating processing executed by the image generating section 12 will be described. The simulation image generating processing is executed using a conventional method as described in, for example, JP laid-open No.2002-209852 A or JP laid-open No.2004-337236.

In a case where the eye's optical characteristic measuring apparatus 2 is the apparatus as described in JP 2002-209852 A, the point spread function (PSF) of the eye to be examined is inputted to the image generating section 12. The image generating section 12 convolves the inputted PSF with image data of a stripe index (intensity distribution data) to generate a simulation image in the case where the stripe index is projected to the fundus of the eye to be examined. The image generating section 12 executes such processing on each of the image data of the 32 stripe indices shown in FIG. 4 to generate 32 simulation images.

Here, in order to shorten a calculation time, the following may be performed. First, the PSF is subjected to Fourier transform to obtain an optical transfer function (OTF). A modulation transfer function (MTF) corresponding to an amplitude component of the OTF is convolved with the image data of each of the stripe indices (which is subjected to Fourier transform). In addition, a phase transfer function (PTF) corresponding to a phase component of the OTF is convolved with the image data of each of the stripe indices (which is subjected to Fourier transform). A result obtained by each convolution is subjected to inverse Fourier transform to obtain a simulation image corresponding to the convolution of the PSF and the image data of the corresponding stripe index. Even in such a case, the processing is executed on each of the image data of the 32 stripe indices.

A process up to the processing for calculating the MTF and the PTF may be executed by the eye's optical characteristic measuring apparatus 2 and the MTF and PTF which are obtained thereby may be inputted to the image generating section 12.

On the other hand, in a case where the eye's optical characteristic measuring apparatus 2 is the apparatus as described in JP laid-open No. 2004-337236, the OTF (MTF and PTF) is inputted to the image generating section 12. That is, the eye's optical characteristic measuring apparatus 2 calculates a pupil diameter of the eye to be examined, total wavefront aberration, Zernike coefficients, and the like and obtains a pupil function based on a result obtained by calculation. Then, the eye's optical characteristic measuring apparatus 2 obtains an OTF (MTF and PTF) of an eyeball of the eye to be examined and the pupil function based on the measuring result and sends the obtained OTF to the ophthalmic examination apparatus 10. The image generating section 12 executes the same processing as that in the above-mentioned case to generate 32 simulation images.

### (Image Display Control Section)

The image display control section 13 corresponds to an example of "image display control means" in the present invention, for causing the display section 17 to display a list of the simulation images generated by the image generating section 12. The image display control section 13 is constructed by including the CPU 100.

The generated 32 simulation images are arranged and displayed by the image display control section 13 in an order based on the contrasts of the eight stripe indices at each of the spatial frequencies of 3, 6, 12, and 18 cycles/diopter. At this time, the sample indices associated with the respective spatial frequencies are also displayed.

At this time, the simulation images and the sample indices which are to be displayed are arranged as shown in, for example, FIG. 4. That is, the simulation images and the sample index images, which are associated with the different spatial frequencies, are arranged in the lateral direction of a display screen of the display section 17 and displayed thereon. In addition, the sample index images associated with the respective spatial frequencies are arranged in the uppermost positions in the longitudinal direction of the display screen and displayed thereon. Then, the simulation images are arranged in descending order from a simulation image associated with an index whose contrast is high and displayed.

Note that FIG. 4 shows the image data of the stripe indices and display objects controlled by the image display control section 13 are the simulation images and the like. Therefore, the displayed sample index images are identical to those shown in FIG. 4. However, each of the simulation images is displayed with a state reflecting the influence of the eye to be examined (eyeball optical system), so that the simulation images are displayed as images different from the original image data of the stripe indices shown in FIG. 4.

The display arrangement of the simulation images is not limited to the above-mentioned arrangement and thus the simulation images may be arranged in ascending order from a simulation image associated with an index whose contrast is low and displayed thereon. The simulation images and the sample index images, which are associated with the different spatial frequencies, may be arranged in the longitudinal direction of the display screen of the display section 17 and displayed thereon. In addition, the sample index images and the simulation images, which are associated with the respective spatial frequencies, may be arranged in the lateral direction of the display screen and displayed thereon. The sample index image is not necessarily located in the uppermost position and thus may be located in a lowermost position or on the right or left side of the display screen.

### (Image Specifying Section)

The image specifying section 14 corresponds to an example of "image specifying means" in the present invention. The image specifying section 14 selects, from the simulation images of the eight stripe indices which are associated with each of the spatial frequencies, simulation images whose contrasts can be recognized by the eye to be examined based on the 32 simulation images generated by the image generating section 12. Then, the image specifying section 14 specifies a simulation image in which a contrast of a stripe index which is the basis for the simulation image in the image generating processing is minimum, from the selected simulation images. The image specifying section 14 is constructed by including the CPU 100. Hereinafter, the image specifying processing executed by the image specifying section 14 will be specifically described.

The image specifying section 14 includes an image analyzing section 14a. The image analyzing section 14a relates to a first stage (image selection processing) of processing performed by the image specifying section 14. The image analyzing section 14a analyzes each of the simulation images generated by the image generating section 12 to obtain a change in density of stripes (intensity value distribution).

FIGS. 5A and 5B show an example of an analyzed pattern of an intensity value distribution of a simulation image. As shown by arrows of FIG. 5A, the image analyzing section 14a obtains the intensity value distribution of the simulation image in a direction in which stripes are arranged.

In a case where a contrast of the simulation image is high (generally, in a case where a contrast of a stripe index which is the basis for the simulation image is high) as shown on the left side of FIG. 5A, a range of change in obtained intensity value distribution widens as shown on the left side of FIG. 5B. Therefore, a difference between a maximum value and a minimum value becomes larger.

On the other hand, in a case where a contrast of the simulation image is low (generally, in a case where a contrast of a stripe index which is the basis for the simulation image is low) as shown on the right side of FIG. 5A, a range of change in obtained intensity value distribution narrows as shown on the right side of FIG. 5B. Therefore, a difference between a maximum value and a minimum value becomes smaller.

The image specifying section 14 compares the amount of change in intensity value (difference between maximum value and minimum value) Δ in the obtained intensity value distribution and the intensity threshold value data 11c (threshold value D). In a case where the amount of change in intensity value is equal to or larger than the intensity threshold value data 11c (Δ ≥ D), the image specifying section 14 determines that the simulation image is a simulation image whose contrast can be recognized by the eye to be examined. On the other hand, in a case where the amount of change in intensity value is smaller than the intensity threshold value data 11c (Δ < D), the image specifying section 14 determines that the simulation image is a simulation image whose contrast cannot be recognized by the eye to be examined.

The image specifying section 14 executes the above-mentioned processing for determining whether or not the contrast can be recognized on each of the 32 simulation images generated by the image generating section 12. Therefore, whether or not the contrast can be recognized by the eye to be examined is determined for each of the 32 simulation images.

The threshold value D set as the intensity threshold value data 11c is, for example, a value suitably set based on a large number of clinical data and the like. Age groups of persons to be examined may be divided and different threshold values may be set to the divided age groups.

The image specifying section 14 specifies a simulation image in which a contrast of a stripe index which is the basis for the simulation image is minimum, from simulation images whose contrasts can be recognized at each of the spatial frequencies (3, 6, 12, and 18 cycles/diopter).

To be more specific, the image specifying section 14 obtains contrast values of stripe indices (which are stored in advance as, for example, the ophthalmic examination data 103b) which are the basis for respective simulation images having been determined that contrasts can be recognized at each of the spatial frequencies, specifies a stripe index having a minimum value among the contrast values, and specifies a simulation image based on the stripe index.

A simulation image can be specified by using the index information instead of actually comparing the contrast values of the stripe indices. As shown in FIG. 4, the eight stripe indices associated with each of the spatial frequencies are provided with index information "circle 1", "circle 2", ..., "circle 8" in descending order from the stripe index whose contrast is highest. The image specifying section 14 can refer to index information of stripe indices which are the basis for respective simulation images having been determined that contrasts can be recognized at each of the spatial frequencies, to thereby specify, as a simulation image whose contrast is minimum, a simulation image based on a stripe index in which a numeral indicated by the index information is largest.

In the case of the above-mentioned processing performed by the image specifying section 14, it should be noted that when a contrast of a stripe index is high, the amount of change in intensity value of a simulation image based on the stripe index becomes larger. The reason is as follows. In view of the fact that the image data of a stripe index and the eye's optical characteristic data of the eye to be examined are convolved with each other to obtain a simulation image, and that the eye's optical characteristic data to be convolved with the respective stripe indices are the same, when the contrast of the stripe index increases, the contrast of the simulation image increases, that is, the amount of change in intensity value becomes larger.

### (Examination Result Creating Section)

The examination result creating section 15 corresponds to "examination result creating means" in the present invention, for adding, onto the examination sheet data 11a, mark information for marking the index information of the stripe indices which are the basis for the simulation images specified in the respective spatial frequencies by the image specifying section 14. The examination result creating section 15 is constructed by including the CPU 100.

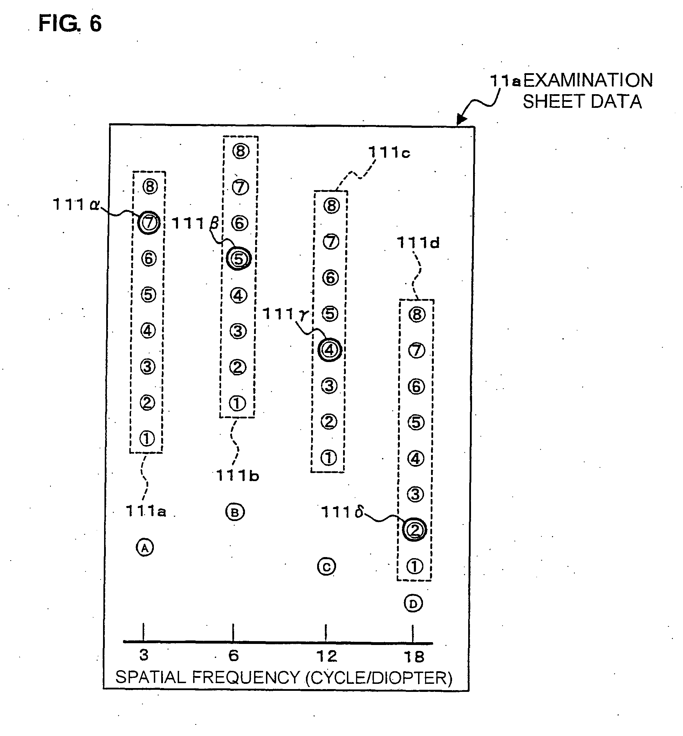

FIG. 6 shows an example of the examination sheet data 11a to which the mark information is added by the examination result creating section 15. Assuming that the simulation images specified by the image specifying section 14 are a simulation image based on a stripe index provided with the index information "circle 7" in the case where the spatial frequency is 3 cycles/diopter, a simulation image based on a stripe index provided with the index information "circle 5" in the case where the spatial frequency is 6 cycles/diopter, a simulation image based on a stripe index provided with the index information "circle 4" in the case where the spatial frequency is 12 cycles/diopter, and a simulation image based on a stripe index provided with the index information "circle 2" in the case where the spatial frequency is 18 cycles/diopter.

Upon receiving the results specified by the image specifying section 14, the examination result creating section 15 adds, to the examination sheet data 11a, mark information 111α for marking the index information "circle 7" of the index information 111a of the eight stripe indices associated with the spatial frequency of 3 cycles/diopter, mark information 111β for marking the index information "circle 5" of the index information 111b of the eight stripe indices associated with the spatial frequency of 6 cycles/diopter, mark information 111γ for marking the index information "circle 4" of the index information 111c of the eight stripe indices associated with the spatial frequency of 12 cycles/diopter, and mark information 111δ for marking the index information "circle 2" of the index information 111d of the eight stripe indices associated with the spatial frequency of 18 cycles/diopter.

Each of the mark information 111α, 111β, 111γ, and 111δ shown in FIG. 6 corresponds to a circle surrounding index information which is a mark target. Note that the "mark information" in the present invention is not limited to such a circle. Therefore, it is possible to apply arbitrary information capable of marking the index information of the stripe index which is the basis for the simulation image specified by the image specifying section 14, and distinguishing the index information from other index information. For example, a display color of the index information of the mark target and a display size thereof can be adjusted.

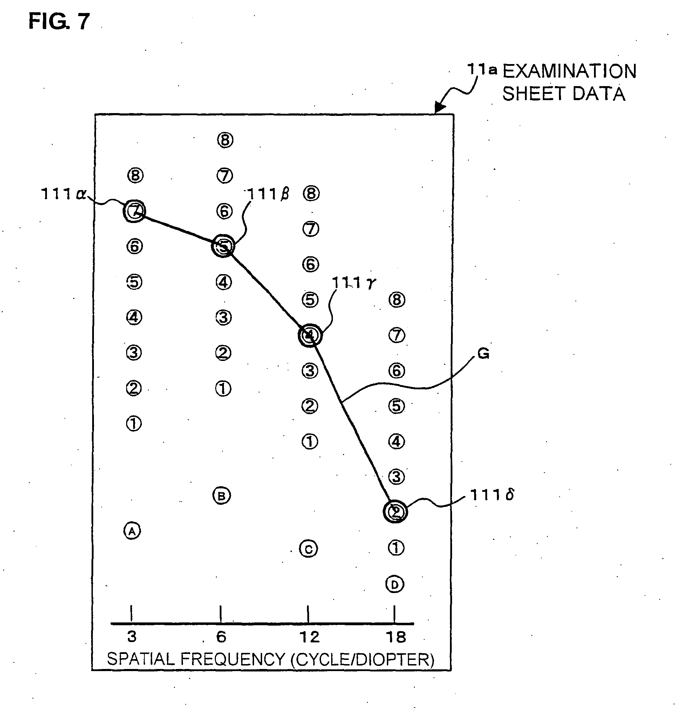

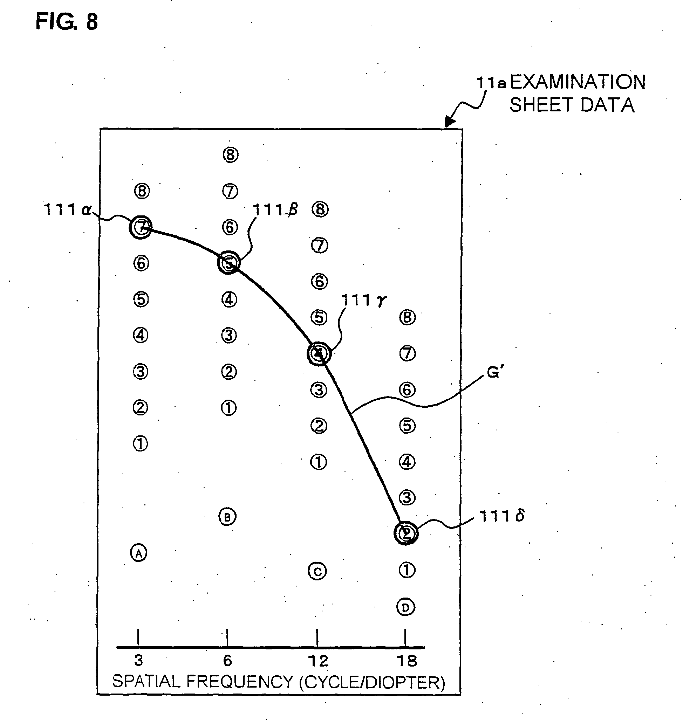

By referring to a graph (see FIG. 7 or 8) described later, index information corresponding to the specified simulation images can be marked (index information intersecting with the graph can be marked as index information corresponding to the specified simulation images so as to be marked after being distinguished from other index information). In a case where the graph is used as the mark information, it is unnecessary to further use the mark information 111α and the like as shown in FIG. 6.

Patterns of the mark information can be set appropriately corresponding to patterns of the index information provided on the examination sheet data 11a. For example, when only numerals "1" to "8" instead of "circle 1" to circle 8" are provided as the index information, it is possible to apply mark information composed of, for example, a circle or a rectangle surrounding the index information of the mark target.

The examination result creating section 15 can add a graph that interconnects the index information corresponding to the simulation images specified by the image specifying section 14 onto the examination sheet data 11a. FIGS. 7 and 8 each show an example of such the graph.

A graph G shown in FIG. 7 is a line graph which is composed of (1) a straight line connecting the index information "circle 7" corresponding to the simulation image specified with regard to the spatial frequency of 3 cycles/diopter and the index information "circle 5" corresponding to the simulation image specified with regard to the spatial frequency of 6 cycles/diopter, (2) a straight line connecting the index information "circle 5" corresponding to the simulation image specified with regard to the spatial frequency of 6 cycles/diopter and the index information "circle 4" corresponding to the simulation image specified with regard to the spatial frequency of 12 cycles/diopter, and (3) a straight line connecting the index information "circle 4" corresponding to the simulation image specified with regard to the spatial frequency of 12 cycles/diopter and the index information "circle 2" corresponding to the simulation image specified with regard to the spatial frequency of 18 cycles/diopter.

A graph G' shown in FIG. 8 is a graph composed of a curve connecting the index information "circle 7" corresponding to the simulation image specified with regard to the spatial frequency of 3 cycles/diopter, the index information "circle 5" corresponding to the simulation image specified with regard to the spatial frequency of 6 cycles/diopter, the index information "circle 4" corresponding to the simulation image specified with regard to the spatial frequency of 12 cycles/diopter, and the index information "circle 2" corresponding to the simulation image specified with regard to the spatial frequency of 18 cycles/diopter. The graph G' is, for example, a graph of an approximated cubic curve passing through the above-mentioned four index information. The graph of the approximated cubic curve is obtained by, for example, the approximation of the MTF by a cubic function (see, for example, "Retina Topography and Wavefront Sensor - Point of Decoding" edited by Naoyuki Maeda, Tetsuro Oshika, and Takashi Fujikado, Medical View Co., Ltd, October 10, 2002).

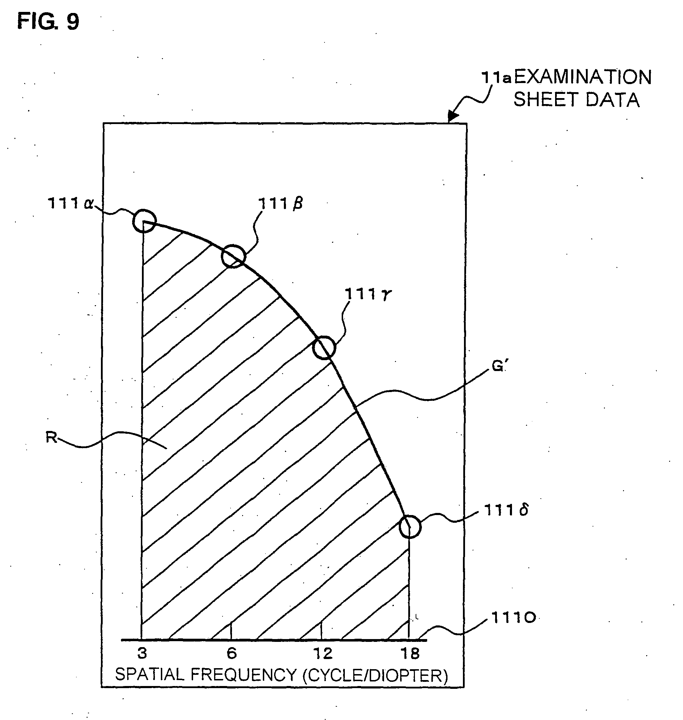

The examination result creating section 15 can calculate an area of a region on the examination sheet data 11a specified by the graph G' shown in FIG. 8 (see, for example, "Retina Topography and Wavefront Sensor - Point of Decoding" edited by Naoyuki Maeda, Tetsuro Oshika, and Takashi Fujikado, Medical View Co., Ltd, October 10, 2002). FIG 9 shows the region on the examination sheet data 11a specified by the graph G' of the approximated cubic curve.

In FIG. 9, a lateral coordinate axis 111O indicates a logarithmic value of a spatial frequency and a longitudinal coordinate axis (not shown) indicates a logarithmic value of contrast sensitivity. The lateral coordinate axis 111O is set in a position in which the logarithmic value of contrast sensitivity in the longitudinal coordinate axis is equal to 0. The logarithm to be used here is a common logarithm.

A region R which is a target for area calculation is a region surrounded on all four sides by (1) the lateral coordinate axis 111O, (2) the graph G', (3) a straight line connecting a position corresponding to a logarithmic value of contrast sensitivity of the stripe index which is the basis for the simulation image specified with regard to the spatial frequency of 3 cycles/diopter (position corresponding to the mark information 111α) and a position corresponding to the spatial frequency of 3 cycles/diopter on the lateral coordinate axis 111O, and (4) a straight line connecting a position corresponding to a logarithmic value of contrast sensitivity of the stripe index which is the basis for the simulation image specified with regard to the spatial frequency of 18 cycles/diopter (position corresponding to the mark information 111δ) and a position corresponding to the spatial frequency of 18 cycles/diopter on the lateral coordinate axis 111O.

An area calculating section 15a performs the normal constant integral calculation to calculate an area of the region R. Here, a function to be integrated is a cubic function expressed by the graph G' and an integration range is from a position (coordinate) corresponding to the spatial frequency of 3 cycles/diopter on the lateral coordinate axis 111O to a position (coordinate) corresponding to the spatial frequency of 18 cycles/diopter thereon.

The area of the region R specified as described above may be called the area under the log contrast sensitivity function (AULCSF) (see, for example, "Retina Topography and Wavefront Sensor - Point of Decoding" edited by Naoyuki Maeda, Tetsuro Oshika, and Takashi Fujikado, Medical View Co., Ltd, October 10, 2002).

### (Examination Result Display Control Section)

The examination result display control section 16 corresponds to an example of "examination result display control means" in the present invention, for causing the display section 17 to display the examination sheet data 11 a to which the mark information (including the above-mentioned graph) are added by the examination result creating section 15. The examination result display control section 16 is constructed by including the CPU 100.

The examination result display control section 16 causes the display section 17 to display a list of the simulation images generated by the image generating section 12 together with the examination sheet data 11 a to which the mark information are added.

Therefore, the examination sheet data 11 a to which the mark information are added as shown in FIG. 6, 7, or 8 and the above-mentioned 32 simulation images are displayed on the display section 17. The sample indices associated with the respective spatial frequencies may be further displayed.

For example, the 32 simulation images (and the sample indices) are arranged and displayed in the same pattern as that in the case of the 32 stripe indices (and sample indices) indicated by the index data 11b shown in FIG. 4. That is, the 32 simulation images are arranged corresponding to the different spatial frequencies in the lateral direction of the display screen of the display section 17 and displayed thereon. In addition, the 32 simulation images are arranged corresponding to the eight stripe indices associated with each of the spatial frequencies in the longitudinal direction of the display screen and displayed thereon.

On the examination sheet data 11a to which the mark information are added, the index information are arranged corresponding to the different spatial frequencies in the lateral direction of the display screen of the display section 17, and the index information of the eight stripe indices associated with each of the spatial frequencies are arranged in the longitudinal direction thereof (see FIGS. 6, 7 and 8).

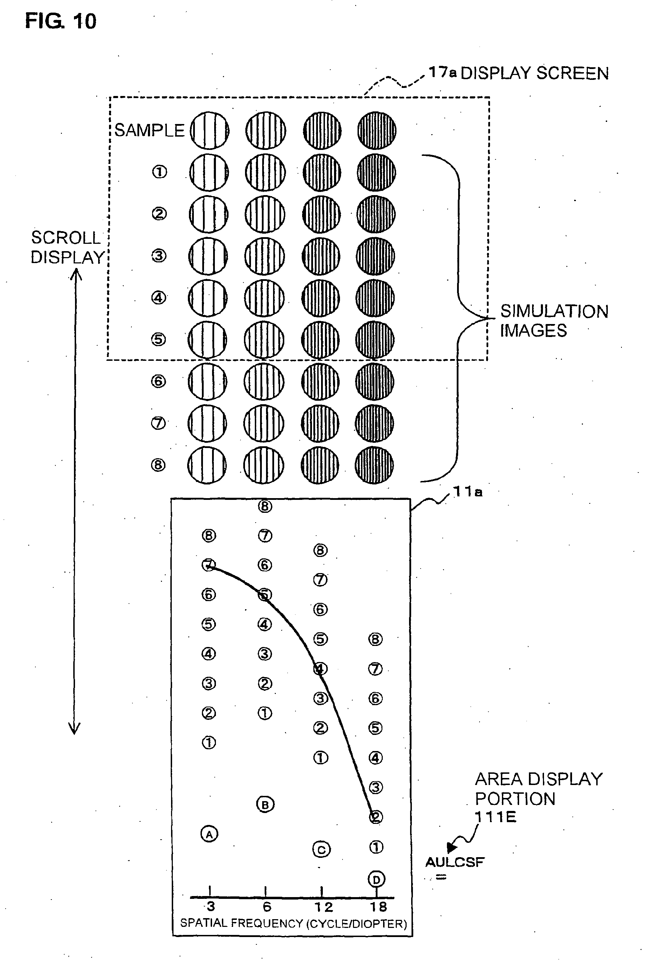

FIG 10 shows an example of a display pattern with respect to the examination sheet data 11 a and the simulation images which are controlled by the examination result display control section 16. According to the display pattern shown in FIG. 10, the sample indices associate with the spatial frequencies of 3, 6, 12 and 18 cycles/diopter are arranged and displayed from the left in an uppermost portion.

The simulation images based on the eight stripe indices associated with each of the spatial frequencies are located below the sample index associated with the corresponding spatial frequency, and arranged and displayed in descending order of contrast (that is, an order of "circle 1", "circle 2",..., "circle 8") in the longitudinal direction.

The examination sheet data 11a to which the mark information (including the graph G') is added is displayed below the simulation images (see FIG. 8). Here, the index information "circle 1" to "circle 8" associated with each of the spatial frequencies are arranged in descending order of contrast sensitivity (that is, an order of "circle 8", "circle 7", ..., "circle 1") in the longitudinal direction and displayed on the examination sheet data 11a. Therefore, a display order of the index information on the examination sheet data 11a is opposite to a display order of the simulation images.

An area display portion 111E for displaying the area of the region R (AULCSF) calculated by the area calculating section 15a of the examination result creating section 15 is provided on the right side of the examination sheet data 11a.

A display content shown in FIG 10 is longitudinally longer than a display screen 17a of the display section 17 (region surrounded by a dot line in FIG. 10). Therefore, a user can operate the mouse 105 or the like to scroll the display content in the longitudinal direction, thereby displaying a desirable part of the display content on the display screen 17a.

The display pattern with respect to the examination sheet data 11 a and the simulation images is not limited to the pattern shown in FIG 10. However, it is desirable that the index information and the simulation images be displayed on the examination sheet data 11 a so as to align the index information with the simulation images.

In other words, the 32 simulation images are arranged corresponding to the different spatial frequencies in one of the longitudinal direction of the display screen 17a and the lateral direction thereof, and arranged corresponding to the eight stripe indices associated with each of the spatial frequencies in the other thereof. The 32 index information of the examination sheet data 11a are arranged corresponding to the different spatial frequencies in the one thereof and arranged corresponding to the eight stripe indices associated with each of the spatial frequencies in the other thereof.

In the display pattern shown in FIG. 10, the 32 simulation images are arranged corresponding to the different spatial frequencies in the lateral direction of the display screen 17a and arranged corresponding to the eight stripe indices associated with each of the spatial frequencies in the longitudinal direction thereof. The 32 index information of the examination sheet data 11 a are arranged corresponding to the different spatial frequencies in the lateral direction of the display screen 17a and arranged corresponding to the eight stripe indices associated with each of the spatial frequencies in the longitudinal direction thereof.

The arrangement direction of the simulation images and the arrangement direction of the index information (the longitudinal direction and the lateral direction) can be reversed. A relative positional relationship between the simulation images and the examination sheet data 11 a is not limited to the longitudinal positional relationship as shown in FIG 10 and thus may be a lateral positional relationship. A window, i.e., screen, for displaying the simulation images and a window for displaying the examination sheet data 11a may be switched therebetween. In this case, a window switching operation can be performed by, for example, a normal window switching operation using the mouse 105 or the like.

### [Operation]

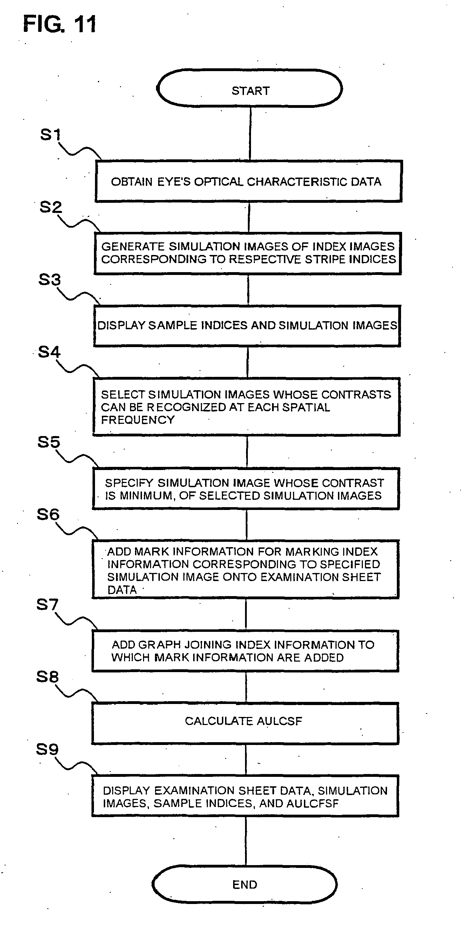

An operation of the ophthalmic examination system 1 having the above-mentioned structure according to this embodiment will be described. FIG 11 is a flow chart showing an example of a mode of the contrast sensitivity examination using the ophthalmic examination system 1. Hereinafter, an example of the operation of the ophthalmic examination system 1 will be described with reference to FIG 11.

First, the eye's optical characteristic data (MTF, PTF, and the like) of the eye to be examined are obtained using the eye's optical characteristic measuring apparatus 2 (Step S1). The obtained eye's optical characteristic data are transmitted to the ophthalmic examination apparatus 10 and inputted to the image generating section 12.

The image generating section 12 generates the simulation image of the index image formed on the fundus of the eye to be examined when each of the 32 stripe indices indicated by the index data 11b is presented to the eye to be examined, based on the inputted eye's optical characteristic data and the index data 11b stored in the data storing section 11 (Step S2). The generated 32 simulation images are sent to each of the image display control section 13 and the image specifying section 14.

The image display control section 13 causes the display section 17 to display a list of the 32 simulation images together with the sample indices (Step S3).

The image specifying section 14 selects simulation images whose contrasts can be recognized by the eye to be examined, from the simulation images of the eight stripe indices which are associated with each of the spatial frequencies, of the 32 simulation images (Step S4). Then, the image specifying section 14 specifies a simulation image in which a contrast of a stripe index which is the basis for the simulation image is minimum, of the simulation images selected in Step S4 at each of the spatial frequencies (Step S5).

Subsequently, the examination result creating section 15 adds mark information for marking index information corresponding to the simulation images specified at the respective spatial frequencies onto the examination sheet data 11a (Step S6). Therefore, in the case of the examination sheet data 11 a, as shown in FIG. 6, the mark information 111 a is added to one of the eight index information associated with the spatial frequency of 3 cycles/diopter. The mark information 111β is added to one of the eight index information associated with the spatial frequency of 6 cycles/diopter. The mark information 111γ is added to one of the eight index information associated with the spatial frequency of 12 cycles/diopter. The mark information 111δ is added to one of the eight index information associated with the spatial frequency of 18 cycles/diopter.

Then, the examination result creating section 15 adds, onto the examination sheet data 11a, a graph joining the index information to which the mark information are added, which are associated with the respective spatial frequencies (Step S7). This graph may be a line graph such as the graph G shown in FIG. 7 or an approximated curve such as the graph G' shown in FIG 8. In this embodiment, the latter graph is used.

The area calculating section 15a of the examination result creating section 15 calculates the area of the region (region R shown in FIG. 9) (AULCSF) which is specified by the approximated curve on the examination sheet data 11a (Step S8).

As shown in FIG. 10, the examination result display control section 16 causes the display section 17 to display the examination sheet data 11a to which the mark information (including the graph G') are added, the 32 simulation images generated in Step S2, the sample indices associated with the respective spatial frequencies, and the area (AULCSF) calculated in Step S8 (Step S9).

### [Operation and Effect]

According to the ophthalmic examination system 1 in this embodiment which performs the above-mentioned operation, the following operations and effects are obtained.

The ophthalmic examination apparatus 10 operates (1) to generate a simulation image of an index image formed on the fundus of the eye to be examined when each of the plurality of stripe indices is presented to the eye to be examined, based on the optical characteristic data of the eye to be examined which are measured by the eye's optical characteristic measuring apparatus 2 and the image data of the stripe indices (index data 11b) associated with each of the spatial frequencies, (2) to select simulation images whose contrasts can be recognized by the eye to be examined, from the simulation images of the plurality of stripe indices which are associated with each of the spatial frequencies, of the generated simulation images, and then to specify a simulation image in which a contrast of a stripe index which is the basis for the simulation image is minimum, from the selected simulation images, (3) to add mark information for marking index information of the stripe index which is the basis for the specified simulation image onto the image data of the examination sheet (examination sheet data 11a), and (4) to display the image data of the examination sheet to which the mark information are added.

According to the ophthalmic examination system 1, the simulation images with respect to the visibility of the stripe indices which are based on the eye's optical characteristic data are used. Therefore, the contrast sensitivity of the eye to be examined can be subjected to a so-called "objective" examination. Thus, the objectivity of the result obtained by the contrast sensitivity examination can be improved as compared with a conventional "subjective" examination based on only a reply from a person to be examined.

According to the contrast sensitivity examination in this embodiment, it is only necessary to measure the optical characteristic data of the eye to be examined. Subsequent processings are automatically performed. Therefore, unlike a conventional case, it is unnecessary that each of a large number of (for example, 32) stripe indices be presented to the eye to be examined to make a reply. Thus, an examination time of the contrast sensitivity examination can be shortened, so that a burden on the person to be examined and an examiner (such as an ophthalmologist) can be reduced.

In the ophthalmic examination system 1 according to this embodiment, the list of the generated simulation images is displayed. Therefore, the examiner can easily grasp the visibility of each of the stripe indices which is caused by the eye to be examined based on the images.

At this time, the listed simulation images are arranged for each of the spatial frequencies associated with the stripe indices which are the basis for the simulation images. In addition, the simulation images are arranged in a stated order of contrast of the stripe indices which are the basis for the simulation images at each of the spatial frequencies. Therefore, the simulation images to be displayed are arranged in an arrangement order (corresponding to an order of use in a subjective examination) of the stripe indices, with the result that association between a simulation image and a stripe index can be easily grasped.

The sample indices associated with the respective spatial frequencies are displayed together with the simulation images, so that a stripe index associated with a spatial frequency, which corresponds to each of the display simulation images can be easily grasped.

In the ophthalmic examination system 1 according to this embodiment, the density of stripes of each of the plurality of generated simulation images is analyzed for each of the spatial frequencies. When the amount of change in density is equal to or larger than the predetermined threshold value (intensity threshold data 11c), it is determined that a contrast of the corresponding simulation image can be recognized by the eye to be examined. Therefore, the contrast sensitivity examination can be suitably automated.

In the ophthalmic examination system 1 according to this embodiment, when the examination sheet data 11a to which the mark information are added is displayed, the simulation images of the respective stripe indices are also displayed. Therefore, the examiner can compare a result automatically obtained by the ophthalmic examination apparatus 10 with the visibility of each of the stripe indices which is caused by the eye to be examined and determine the validity of the result by the examiner himself or herself.

At this time, the index information and the simulation images are displayed on the examination sheet data 11a so as to align the index information with the simulation images, so that association between a simulation image and a stripe index can be easily grasped.

In the ophthalmic examination system 1 according to this embodiment, the graph joining the index information corresponding to the specified simulation images can be added onto the examination sheet data 11 a. Therefore, the examiner can easily grasp a state of a change in the result obtained by the examination which is caused with a change in spatial frequency. In particular, when the approximated graph as shown in FIG 8 is used, the AULCSF can be automatically calculated.

### [Modified Examples]

Various modified examples of the ophthalmic examination system 1 according to this embodiment will be described.

When a conventional "subjective" examination is performed in addition to the "objective" contrast sensitivity examination using the ophthalmic examination system 1, a result obtained by the objective examination can be compared with a result obtained by the subjective examination. Therefore, it is possible to determine, for example, the presence or absence of abnormality of the nervous system of the person to be examined.

For example, there is a case where the result obtained by the objective examination is preferable but the result obtained by the subjective examination is not preferable. In this case, there is little problem on an eyeball optical system of the eye to be examined but there is a problem on the "visibility" caused by the brain of the person to be examined.

Therefore, it is possible to suspect any abnormality of the nervous system of the person to be examined. Thus, for example, an examination for checking the presence or absence of the abnormality can be continuously performed. When such an examination method is employed, the person to be examined can be diagnosed from various perspectives, so that the accuracy of diagnosis can be improved.

The objective result obtained by the ophthalmic examination system 1 can be used for a conventional subjective examination.

For example, before the subjective contrast sensitivity examination is performed, the objective examination is performed using the ophthalmic examination system 1 to obtain a result obtained by objective examination of contrast sensitivity of the eye to be examined. In other words, objective data with respect to a stripe index, whose contrast can be recognized, of the plurality of stripe indices associated with each of the spatial frequencies, are obtained.

The obtained objective result is used to select an examination start index for the subjective examination. For example, as shown in FIG 10, assume that the stripe indices provided with the index information "circle 7", "circle 5", "circle 4", and "circle 2" at the respective spatial frequencies of 3, 6, 12, and 18 cycles/diopter are obtained as results obtained by objective examination. In this case, the subjective examination is performed as follows. The stripe index provided with the index information "circle 7" is first presented at the spatial frequency of 3 cycles/diopter. The stripe index provided with the index information "circle 5" is first presented at the spatial frequency of 6 cycles/diopter. The stripe index provided with the index information "circle 4" is first presented at the spatial frequency of 12 cycles/diopter. The stripe index provided with the index information "circle 2" is first presented at the spatial frequency of 18 cycles/diopter.

As described above, when the result obtained by the objective examination is used for the subjective examination, it is unnecessary to present the stripe indices in order from a stripe index whose contrast is maximum (index information "circle 1 "), unlike a conventional case. Therefore, the examination time of the subjective examination can be shortened. That is, an approximate estimated contrast which can be recognized by the eye to be examined can be obtained by the objective examination, so a stripe index having the estimated contrast is first presented. Thus, the subjective examination can be performed in a short time as compared with a conventional case.

The examination methods described in the modified examples can be executed as appropriate for an ophthalmic examination system according to a second embodiment as described later.

### [With respect to Ophthalmic Examination Program]

The operation of the ophthalmic examination apparatus 10 described in this embodiment is executed by the CPU 100 based on the ophthalmic examination program 103a stored in the hard disk drive 103. The ophthalmic examination program 103a corresponds to an example of an "ophthalmic examination program" according to the present invention.

The ophthalmic examination program 103a can be stored in an arbitrary recording medium so as to be readable by a computer. The recording medium which can be used as appropriate is a medium capable of recording data using an arbitrary physical method including an electrical method, a magnetic method, and an optical method, such as a floppy (registered trademark) disk, a CD-ROM, a CD-R(W), a DVD-ROM, a DVD-R(W), a DVD-RAM, an MO, or any memory card.

When the ophthalmic examination program 103a is stored in a server or a storage device which is located on a network such as a LAN or the Internet, the ophthalmic examination apparatus 10 can be constructed so as to download the ophthalmic examination program 103a to be used through the network.

### <Second Embodiment>

An ophthalmic examination system according to another embodiment of the present invention will be described.

In the first embodiment, the contrast sensitivity examination is automated. In contrast to this, in a second embodiment described below, a part of the processing performed by the ophthalmic examination apparatus 10 according to the first embodiment is manually performed by a user.

### [Structure]

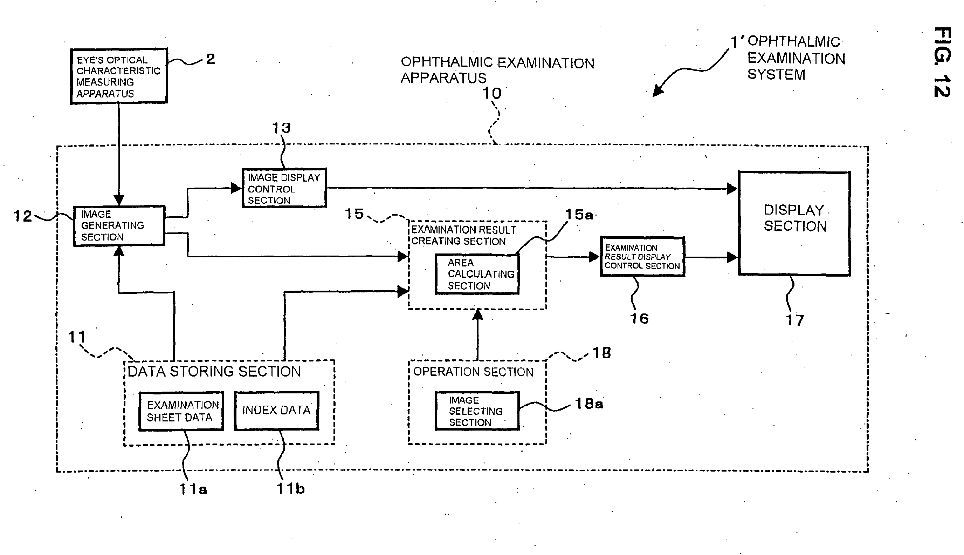

FIG. 12 shows an example of the entire structure of the ophthalmic examination system according to this embodiment. In FIG. 12, the same constituent sections as those in the first embodiment are indicated by the same reference symbols. As in the first embodiment, an ophthalmic examination system 1' includes the eye's optical characteristic measuring apparatus 2 and the ophthalmic examination apparatus 10. The ophthalmic examination apparatus 10 has the same hardware structure as that in the first embodiment (see FIG. 2).

The ophthalmic examination apparatus 10 according to this embodiment includes not the image specifying section 14 described in the first embodiment but an operation section 18. The operation section 18 includes a device such as the keyboard 104 or the mouse 105 as shown in FIG. 2. A device such as a trackball, a joystick, a touch panel LCD, or an ophthalmic examination control panel may be included. The operation section 18 corresponds to an example of "operating means" in the present invention.

The operation section 18 includes an image selecting section 18a. The image selecting section 18a corresponds to an example of "image selecting means" in the present invention and operated by a user to select a simulation image for each spatial frequency from the simulation images displayed on the display section 17 by the image display control section 13. The image selecting section 18a is constructed by, for example, the mouse 105.

To be more specific, the user visually checks contrast states of the displayed simulation images and selects simulation images whose contrasts may be recognized by the eye to be examined from the simulation images of the plurality of indices at each spatial frequency. Then, the user specifies a simulation image in which a contrast of a stripe index which is the basis for the simulation image is minimum, from the selected simulation images at each spatial frequency. A mouse pointer is positioned on the simulation image specified for each spatial frequency and the simulation image is clicked. As described above, the image selecting section 18a corresponds to an example of the "image selecting means" in the present invention.

A result obtained by selecting the simulation image by the image selecting section 18 is inputted to the examination result creating section 15.

### [Operation]

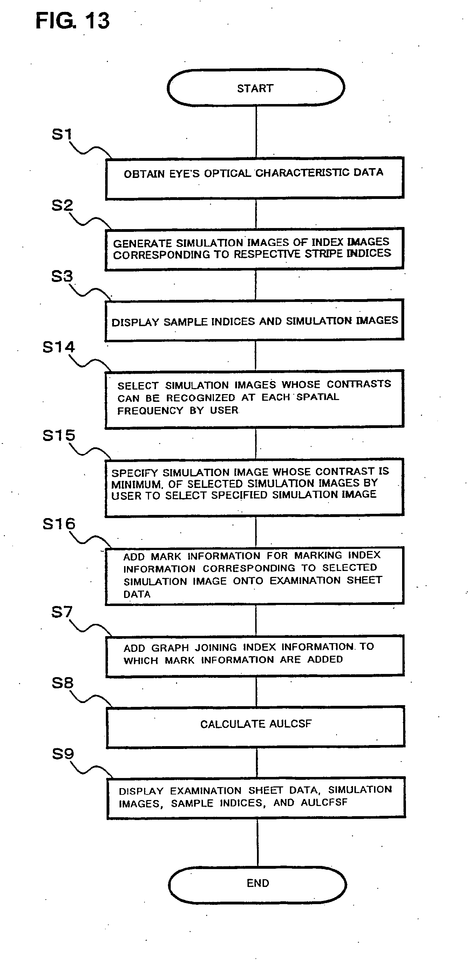

An operation of the ophthalmic examination system 1' according to this embodiment will be described. FIG 13 is a flow chart showing an example of a mode of a contrast sensitivity examination using the ophthalmic examination system 1'. Hereinafter, an example of the operation of the ophthalmic examination system 1' will be described with reference to FIG. 13.

In the flow chart of FIG. 13, the same steps as the steps executed in the first embodiment will be described with reference to the same step numbers as those in the flow chart of FIG. 11.

As in the first embodiment, the eye's optical characteristic data (MTF, PTF, and the like) of the eye to be examined are obtained using the eye's optical characteristic measuring apparatus 2 (Step S1). A simulation image of an index image formed on the fundus of the eye to be examined when each of the stripe indices is presented to the eye to be examined is generated by the image generating section 12 of the ophthalmic examination apparatus 10 (Step S2). A list of the generated simulation images is displayed on the display section 17 together with the sample indices by the image display control section 13 (Step S3).

The user visually checks the contrast states of the displayed simulation images and selects simulation images whose contrasts may be recognized by the eye to be examined from the simulation images of the plurality of indices at each spatial frequency (Step S 14).

Then, the user specifies a simulation image in which a contrast of a stripe index which is the basis for the simulation image is minimum, of the simulation images selected in Step S 14 at each spatial frequency and operates the image selecting section 18a to select the specified simulation images (Step S 15).

Subsequently, the examination result creating section 15 adds mark information for marking index information corresponding to the simulation images selected for the respective spatial frequencies onto the examination sheet data 11a (Step S16).

Then, the examination result creating section 15 adds, onto the examination sheet data 11 a, a graph joining the index information to which the mark information are added, which are associated with the respective spatial frequencies (Step S7). The area calculating section 15a calculates AULCSF (Step S8).

The examination result display control section 16 causes the display section 17 to display the examination sheet data 11a to which the mark information are added, the simulation images generated in Step S2, the sample indices associated with the respective spatial frequencies, and the AULCSF calculated in Step S8 (Step S9). An example of the display pattern is shown in FIG 10.

### [Operation and Effect]

According to the ophthalmic examination system 1' in this embodiment which performs the above-mentioned operation, the following operations and effects are obtained.

As in the first embodiment, the ophthalmic examination apparatus 10 of the ophthalmic examination system 1' generates a simulation image of an index image formed on the fundus of the eye to be examined when each of the stripe indices is presented to the eye to be examined, based on the optical characteristic data of the eye to be examined which are measured by the eye's optical characteristic measuring apparatus 2 and the image data of the plurality of stripe indices (index data 11b) associated with each of the spatial frequencies.

Here, in this embodiment, the user (examiner) selects simulation images whose contrasts can be recognized by the eye to be examined, from the simulation images of the plurality of stripe indices which are associated with each of the spatial frequencies, of the generated simulation images. Then, the user determines a simulation image to be specified in which a contrast of a stripe index which is the basis for the simulation image is minimum, from the selected simulation images, to select the specified simulation image.

The ophthalmic examination apparatus 10 operates to add the mark information for marking the index information of the stripe index which is the basis for the simulation image selected by the user onto the examination sheet data 11 a and to display the examination sheet data 11 a to which the mark information are added.

Therefore, as in the case of the first embodiment, when the simulation images with respect to the visibility of the stripe indices which are based on the eye's optical characteristic data are used, the contrast sensitivity of the eye to be examined can be objectively examined. Thus, the objectivity of the result obtained by examination can be improved.

Further, a part of the contrast sensitivity examination can be automated, so an examination time thereof can be shortened. Therefore, a burden on the person to be examined and the examiner can be reduced.

### <Other Modified Examples>

The first and second embodiments described above are structural examples for suitably embodying the present invention. Therefore, for example, the following various modifications can be made as appropriate.

In the above-mentioned embodiments, the stripe index having vertical stripes is used. A stripe index having horizontal stripes or a stripe index having oblique stripes may also be used. Plural kinds of stripe indices whose stripe arrangement directions are different from one another may be used. For example, two kinds of index data 11b including vertical stripe indices (stripe arrangement direction is the lateral direction) and horizontal stripe indices (stripe arrangement direction is the longitudinal direction) are stored in advance. Therefore, the examination can be performed using one kind of stripe indices or both kinds of stripe indices.

It may be desirable to perform the examination using stripe indices whose stripe arrangement directions are different from one another in a case where, for example, both the vertical stripe indices and the horizontal stripe indices are used. In a conventional case, it is difficult to perform such the examination because the examination takes a long time. However, according to the present invention, the examination time can be shortened, so that the examination can be performed using plural kinds of stripe indices.

In the above-mentioned embodiments, the structure using the stripe indices as indices for contrast sensitivity examination is described. It is also possible to employ a structure using another index chart such as an ETDRS chart including alphabets and Landolt rings.

## Claims

1. An ophthalmic examination program for operating a computer comprising storing means for prestoring image data of a predetermined examination sheet in which index information indicating a plurality of indices whose contrasts are different from one another at each of at least two different spatial frequencies and image data of the plurality of indices associated with the at least two spatial frequencies are arranged and display means,
wherein the ophthalmic examination program causes the computer to function as:
image generating means for generating a simulation image of an index image formed on a fundus of an eye to be examined when each of the plurality of indices is presented to the eye to be examined is generated based on optical characteristic data of the eye to be examined and image data of the plurality of indices associated with the at least two spatial frequencies;
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images;
examination result creating means for adding mark information for making index information of the index which is the basis for the specified simulation image onto the image data of the predetermined examination sheet; and
examination result display control means for causing the display means to display the image data of the examination sheet to which the mark information is added.

2. An ophthalmic examination program according to claim 1, wherein the ophthalmic examination program causes the computer to further function as image display control means for causing the display means to display a list of the simulation images of the plurality of indices which are associated with each of the at least two spatial frequencies, which are generated by the image generating means.

3. An ophthalmic examination program according to claim 2, wherein the ophthalmic examination program causes the image display control means to arrange the generated simulation images in an order of contrast of the plurality of indices associated with each of the at least two spatial frequencies to display a list of the generated simulation images.

4. An ophthalmic examination program according to claim 2 or 3, wherein the ophthalmic examination program causes the image display control means to display a predetermined sample index associated with each of the at least two spatial frequencies together with the simulation images of the plurality of indices.

5. An ophthalmic examination program according to any one of claims 2 to 4, wherein:
the computer further comprises operating means;
each of the plurality of indices associated with each of the at least two spatial frequencies comprises a stripe index including stripes whose density sinusoidally changes;
the ophthalmic examination program causes the operating means to function as image selecting means included in the image specifying means, for selecting a simulation image at each of the at least two spatial frequencies, from the displayed list of the simulation images by a user; and
the ophthalmic examination program causes the examination result creating means to add mark information for marking the selected simulation image to the image data of the examination sheet.

6. An ophthalmic examination program according to any one of claims 1 to 4, wherein:
each of the plurality of indices associated with each of the at least two spatial frequencies comprises a stripe index including stripes whose density sinusoidally changes; and
the ophthalmic examination program causes the image specifying means to analyze the density of the stripes in the simulation image of each of the plurality of indices associated with each of the at least two spatial frequencies and to select the corresponding simulation image as a simulation image whose contrast can be recognized when an amount of change in density is equal to or larger than a predetermined threshold value.

7. An ophthalmic examination program according to claim 1, wherein the ophthalmic examination program causes the examination result display control means to cause the display means to display a list of the simulation images of the plurality of indices which are associated with each of the at least two spatial frequencies, which are generated by the image generating means, together with the image data of the examination sheet to which the mark information is added.

8. An ophthalmic examination program according to claim 7, wherein:
the simulation images to be displayed are arranged corresponding to different spatial frequencies in one of a longitudinal direction and a lateral direction on a display screen of the display means and arranged corresponding to the plurality of indices associated with each of the spatial frequencies in the other of the longitudinal direction and the lateral direction; and
the index information to be displayed on the image data of the examination sheet to which the mark information is added are arranged corresponding to the different spatial frequencies in the one of the longitudinal direction and the lateral direction and arranged corresponding to the plurality of indices associated with each of the spatial frequencies in the other of the longitudinal direction and the lateral direction.

9. An ophthalmic examination program according to claim 8, wherein the index information on the image data of the examination sheet are arranged in the other of the longitudinal direction and the lateral direction in an order of contrast sensitivities which are reciprocals of contrast values of the plurality of indices.

10. An ophthalmic examination program according to any one of claims 1 to 9, wherein the mark information added onto the image data of the examination sheet by the examination result creating means comprises a graph joining index information associated with the at least two spatial frequencies.

11. An ophthalmic examination program according to claim 10, wherein the ophthalmic examination program causes the examination result creating means to calculate an area of a region which is specified by the graph and located on the image data of the predetermined examination sheet.

12. An ophthalmic examination apparatus connected to an eye's optical characteristic measuring apparatus for obtaining optical characteristic data of an eye to be examined, wherein the ophthalmic examination apparatus comprises:
storing means for prestoring image data of a predetermined examination sheet in which index information indicating a plurality of indices whose contrasts are different from one another at each of at least two different spatial frequencies and image data of the plurality of indices associated with the at least two spatial frequencies are arranged;
display means;
image generating means for generating a simulation image of an index image formed on a fundus of an eye to be examined when each of the plurality of indices is presented to the eye to be examined is generated based on optical characteristic data of the eye to be examined and image data of the plurality of indices associated with the at least two spatial frequencies;
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images;
examination result creating means for adding mark information for making index information of the index which is the basis for the specified simulation image onto the image data of the predetermined examination sheet; and
examination result display control means for causing the display means to display the image data of the predetermined examination sheet to which the mark information is added.

13. An ophthalmic examination system, comprising:
an eye's ophthalmic characteristic measuring apparatus for obtaining optical characteristic data; and
an ophthalmic examination apparatus,
wherein the ophthalmic examination apparatus comprises:
storing means for prestoring image data of a predetermined examination sheet in which index information indicating a plurality of indices whose contrasts are different from one another at each of at least two different spatial frequencies and image data of the plurality of indices associated with the at least two spatial frequencies are arranged;
display means;
image generating means for generating a simulation image of an index image formed on a fundus of an eye to be examined when each of the plurality of indices is presented to the eye to be examined is generated based on optical characteristic data, obtained by the eye's optical characteristic measuring apparatus, of the eye to be examined and image data of the plurality of indices associated with the at least two spatial frequencies;
image specifying means for selecting simulation images whose contrasts can be recognized by the eye to be examined, from the simulation images of the plurality of indices which are associated with each of the at least two spatial frequencies, of the generated simulation images and specifying a simulation image in which a contrast of an index which is a basis for the simulation image is minimum, from the selected simulation images;
examination result creating means for adding mark information for making index information of the index which is the basis for the specified simulation image onto the image data of the predetermined examination sheet; and
examination result display control means for causing the display means to display the image data of the examination sheet to which the mark information is added.
